# EUROPEAN PATENT APPLICATION

(11) **EP 3 202 889 A1**
(43) Date of publication of application: **09.08.2017**
(21) Application number: 15846038.6
(22) Date of filing: 19.08.2015
(51) Int. Cl.: C12N 1/00, C12M 1/00, C12M 3/00, C12N 5/07, C12N 5/0735, C12N 5/10

(54) **CELL CULTURE MEDIUM AND CULTURE METHOD USING SAME**

(30) Priority: 29.09.2014 JP 2014197922
(71) Applicant: Kaneka Corporation, Osaka-shi, Osaka 530-8288 (JP); National Hospital Organization, Tokyo 152-8621 (JP)
(72) Inventor: KATO Tomohisa, Takasago-shi Hyogo 676-8688 (JP); KANEMURA Yonehiro, Osaka-shi Osaka 540-0006 (JP); SHOFUDA Tomoko, Osaka-shi Osaka 540-0006 (JP); FUKUSUMI Hayato, Osaka-shi Osaka 540-0006 (JP)
(74) Representative: Hoffmann Eitle
(86) International application number: PCT/JP2015/073224
(87) International publication number: WO 2016/051983

(57) **Abstract**

It is an object of the present invention to provide a cell culture medium capable of enhancing cell growth efficiency without using feeder cells, in particular wherein the cell culture medium does not comprise serum. According to the present invention, a cell culture medium comprising fibrin 5 and Zeta polypeptide is provided.

SEQUENCE LISTING
<110>  Kaneka Corporation
<120>  A medium for cell culture and a method for culture using it
<130>  151269A
<160>  4
<170>  PatentIn version 3.5
<210>  1
<211>  465
<212>  PRT
<213>  human
<400>  1
Met Phe Val Trp Asp Ser His Arg Ser Phe Lys Pro Met Arg Ser Gly
1               5                   10                  15
Asn Cys Arg Gln Arg Tyr Met Ile Leu Thr Val Thr Ile Leu Ala Leu
            20                  25                  30
Cys Leu Pro Ser Pro Gly Asn Ala Gln Ala Gln Cys Thr Asn Gly Phe
        35                  40                  45
Asp Leu Asp Arg Gln Ser Gly Gln Cys Leu Asp Ile Asp Glu Cys Arg
    50                  55                  60
Thr Ile Pro Glu Ala Cys Arg Gly Asp Met Met Cys Val Asn Gln Asn
65                  70                  75                  80
Gly Gly Tyr Leu Cys Ile Pro Arg Thr Asn Pro Val Tyr Arg Gly Pro
                85                  90                  95
Tyr Ser Asn Pro Tyr Ser Thr Pro Tyr Ser Gly Pro Tyr Pro Ala Ala
            100                 105                 110
Ala Pro Pro Leu Ser Ala Pro Asn Tyr Pro Thr Ile Ser Arg Pro Leu
        115                 120                 125
Ile Cys Arg Phe Gly Tyr Gln Met Asp Glu Ser Asn Gln Cys Val Asp
    130                 135                 140
Val Asp Glu Cys Ala Thr Asp Ser His Gln Cys Asn Pro Thr Gln Ile
145                 150                 155                 160
Cys Ile Asn Thr Glu Gly Gly Tyr Thr Cys Ser Cys Thr Asp Gly Tyr
                165                 170                 175
Trp Leu Leu Glu Gly Gln Cys Leu Asp Ile Asp Glu Cys Arg Tyr Gly
            180                 185                 190
Tyr Cys Gln Gln Leu Cys Ala Asn Val Pro Gly Ser Tyr Ser Cys Thr
        195                 200                 205
Cys Asn Pro Gly Phe Thr Leu Asn Glu Asp Gly Arg Ser Cys Gln Asp
    210                 215                 220
Val Asn Glu Cys Ala Thr Glu Asn Pro Cys Val Gln Thr Cys Val Asn
225                 230                 235                 240
Thr Tyr Gly Ser Phe Ile Cys Arg Cys Asp Pro Gly Tyr Glu Leu Glu
                245                 250                 255
Glu Asp Gly Val His Cys Ser Asp Met Asp Glu Cys Ser Phe Ser Glu
            260                 265                 270
Phe Leu Cys Gln His Glu Cys Val Asn Gln Pro Gly Thr Tyr Phe Cys
        275                 280                 285
Ser Cys Pro Pro Gly Tyr Ile Leu Leu Asp Asp Asn Arg Ser Cys Gln
    290                 295                 300
Asp Ile Asn Glu Cys Glu His Arg Asn His Thr Cys Asn Leu Gln Gln
305                 310                 315                 320
Thr Cys Tyr Asn Leu Gln Gly Gly Phe Lys Cys Ile Asp Pro Ile Arg
                325                 330                 335
Cys Glu Glu Pro Tyr Leu Arg Ile Ser Asp Asn Arg Cys Met Cys Pro
            340                 345                 350
Ala Glu Asn Pro Gly Cys Arg Asp Gln Pro Phe Thr Ile Leu Tyr Arg
        355                 360                 365
Asp Met Asp Val Val Ser Gly Arg Ser Val Pro Ala Asp Ile Phe Gln
    370                 375                 380
Met Gln Ala Thr Thr Arg Tyr Pro Gly Ala Tyr Tyr Ile Phe Gln Ile
385                 390                 395                 400
Lys Ser Gly Asn Glu Gly Arg Glu Phe Tyr Met Arg Gln Thr Gly Pro
                405                 410                 415

Ile Ser Ala Thr Leu Val Met Thr Arg Pro Ile Lys Gly Pro Arg Glu
            420                 425                 430
Ile Gln Leu Asp Leu Glu Met Ile Thr Val Asn Thr Val Ile Asn Phe
        435                 440                 445
Arg Gly Ser Ser Val Ile Arg Leu Arg Ile Tyr Val Ser Gln Tyr Pro
    450                 455                 460
Phe
465
<210> 2
<211> 461
<212> PRT
<213> mouse
<400> 2
Met Gly Pro Arg Ser Phe Glu Pro Met His Ser Gly Leu Cys Arg Gln
1               5                   10                  15
Arg Arg Met Ile Leu Thr Val Thr Ile Leu Ala Leu Trp Leu Pro His
            20                  25                  30
Pro Gly Asn Ala Gln Gln Gln Cys Thr Asn Gly Phe Asp Leu Asp Arg
        35                  40                  45
Gln Ser Gly Gln Cys Leu Asp Ile Asp Glu Cys Arg Thr Ile Pro Glu
    50                  55                  60
Ala Cys Arg Gly Asp Met Met Cys Val Asn Gln Asn Gly Gly Tyr Leu
65                  70                  75                  80
Cys Ile Pro Arg Thr Asn Pro Val Tyr Arg Gly Pro Tyr Ser Asn Pro
                85                  90                  95
Tyr Ser Thr Ser Tyr Ser Gly Pro Tyr Pro Ala Ala Ala Pro Pro Val
            100                 105                 110
Pro Ala Ser Asn Tyr Pro Thr Ile Ser Arg Pro Leu Val Cys Arg Phe
        115                 120                 125
Gly Tyr Gln Met Asp Glu Gly Asn Gln Cys Val Asp Val Asp Glu Cys
    130                 135                 140
Ala Thr Asp Ser His Gln Cys Asn Pro Thr Gln Ile Cys Ile Asn Thr
145                 150                 155                 160
Glu Gly Gly Tyr Thr Cys Ser Cys Thr Asp Gly Tyr Trp Leu Leu Glu
                165                 170                 175
Gly Gln Cys Leu Asp Ile Asp Glu Cys Arg Tyr Gly Tyr Cys Gln Gln
            180                 185                 190
Leu Cys Ala Asn Val Pro Gly Ser Tyr Ser Cys Thr Cys Asn Pro Gly
        195                 200                 205
Phe Thr Leu Asn Asp Asp Gly Arg Ser Cys Gln Asp Val Asn Glu Cys
    210                 215                 220
Glu Thr Glu Asn Pro Cys Val Gln Thr Cys Val Asn Thr Tyr Gly Ser
225                 230                 235                 240
Phe Ile Cys Arg Cys Asp Pro Gly Tyr Glu Leu Glu Glu Asp Gly Ile
                245                 250                 255
His Cys Ser Asp Met Asp Glu Cys Ser Phe Ser Glu Phe Leu Cys Gln
            260                 265                 270
His Glu Cys Val Asn Gln Pro Gly Ser Tyr Phe Cys Ser Cys Pro Pro
        275                 280                 285
Gly Tyr Val Leu Leu Asp Asp Asn Arg Ser Cys Gln Asp Ile Asn Glu
    290                 295                 300
Cys Glu His Arg Asn His Thr Cys Thr Ser Leu Gln Thr Cys Tyr Asn
305                 310                 315                 320
Leu Gln Gly Gly Phe Lys Cys Ile Asp Pro Ile Ser Cys Glu Glu Pro
                325                 330                 335
Tyr Leu Leu Ile Gly Glu Asn Arg Cys Met Cys Pro Ala Glu His Thr
            340                 345                 350
Ser Cys Arg Asp Gln Pro Phe Thr Ile Leu Tyr Arg Asp Met Asp Val
        355                 360                 365
Val Ser Gly Arg Ser Val Pro Ala Asp Ile Phe Gln Met Gln Ala Thr
    370                 375                 380
Thr Arg Tyr Pro Gly Ala Tyr Tyr Ile Phe Gln Ile Lys Ser Gly Asn
385                 390                 395                 400

Glu Gly Arg Glu Phe Tyr Met Arg Gln Thr Gly Pro Ile Ser Ala Thr
                405                 410                 415
Leu Val Met Thr Arg Pro Ile Lys Gly Pro Arg Asp Ile Gln Leu Asp
            420                 425                 430
Leu Glu Met Ile Thr Val Asn Thr Val Ile Asn Phe Arg Gly Ser Ser
        435                 440                 445
Val Ile Arg Leu Arg Ile Tyr Val Ser Gln Tyr Pro Phe
    450                 455                 460
<210>  3
<211>  245
<212>  PRT
<213>  human
<400>  3
Met Asp Lys Asn Glu Leu Val Gln Lys Ala Lys Leu Ala Glu Gln Ala
1               5                   10                  15
Glu Arg Tyr Asp Asp Met Ala Ala Cys Met Lys Ser Val Thr Glu Gln
            20                  25                  30
Gly Ala Glu Leu Ser Asn Glu Glu Arg Asn Leu Leu Ser Val Ala Tyr
        35                  40                  45
Lys Asn Val Val Gly Ala Arg Arg Ser Ser Trp Arg Val Val Ser Ser
    50                  55                  60
Ile Glu Gln Lys Thr Glu Gly Ala Glu Lys Lys Gln Gln Met Ala Arg
65                  70                  75                  80
Glu Tyr Arg Glu Lys Ile Glu Thr Glu Leu Arg Asp Ile Cys Asn Asp
                85                  90                  95
Val Leu Ser Leu Leu Glu Lys Phe Leu Ile Pro Asn Ala Ser Gln Ala
            100                 105                 110
Glu Ser Lys Val Phe Tyr Leu Lys Met Lys Gly Asp Tyr Tyr Arg Tyr
        115                 120                 125
Leu Ala Glu Val Ala Ala Gly Asp Asp Lys Lys Gly Ile Val Asp Gln
    130                 135                 140
Ser Gln Gln Ala Tyr Gln Glu Ala Phe Glu Ile Ser Lys Lys Glu Met
145                 150                 155                 160
Gln Pro Thr His Pro Ile Arg Leu Gly Leu Ala Leu Asn Phe Ser Val
                165                 170                 175
Phe Tyr Tyr Glu Ile Leu Asn Ser Pro Glu Lys Ala Cys Ser Leu Ala
            180                 185                 190
Lys Thr Ala Phe Asp Glu Ala Ile Ala Glu Leu Asp Thr Leu Ser Glu
        195                 200                 205
Glu Ser Tyr Lys Asp Ser Thr Leu Ile Met Gln Leu Leu Arg Asp Asn
    210                 215                 220
Leu Thr Leu Trp Thr Ser Asp Thr Gln Gly Asp Glu Ala Glu Ala Gly
225                 230                 235                 240
Glu Gly Gly Glu Asn
                245
<210>  4
<211>  245
<212>  PRT
<213>  mouse
<400>  4
Met Asp Lys Asn Glu Leu Val Gln Lys Ala Lys Leu Ala Glu Gln Ala
1               5                   10                  15
Glu Arg Tyr Asp Asp Met Ala Ala Cys Met Lys Ser Val Thr Glu Gln
            20                  25                  30
Gly Ala Glu Leu Ser Asn Glu Glu Arg Asn Leu Leu Ser Val Ala Tyr
        35                  40                  45
Lys Asn Val Val Gly Ala Arg Arg Ser Ser Trp Arg Val Val Ser Ser
    50                  55                  60
Ile Glu Gln Lys Thr Glu Gly Ala Glu Lys Lys Gln Gln Met Ala Arg
65                  70                  75                  80
Glu Tyr Arg Glu Lys Ile Glu Thr Glu Leu Arg Asp Ile Cys Asn Asp
                85                  90                  95
Val Leu Ser Leu Leu Glu Lys Phe Leu Ile Pro Asn Ala Ser Gln Pro

                100                 105                 110
Glu Ser Lys Val Phe Tyr Leu Lys Met Lys Gly Asp Tyr Tyr Arg Tyr
        115                 120                 125
Leu Ala Glu Val Ala Ala Gly Asp Asp Lys Lys Gly Ile Val Asp Gln
    130                 135                 140
Ser Gln Gln Ala Tyr Gln Glu Ala Phe Glu Ile Ser Lys Lys Glu Met
145                 150                 155                 160
Gln Pro Thr His Pro Ile Arg Leu Gly Leu Ala Leu Asn Phe Ser Val
                165                 170                 175
Phe Tyr Tyr Glu Ile Leu Asn Ser Pro Glu Lys Ala Cys Ser Leu Ala
            180                 185                 190
Lys Thr Ala Phe Asp Glu Ala Ile Ala Glu Leu Asp Thr Leu Ser Glu
        195                 200                 205
Glu Ser Tyr Lys Asp Ser Thr Leu Ile Met Gln Leu Leu Arg Asp Asn
    210                 215                 220
Leu Thr Leu Trp Thr Ser Asp Thr Gln Gly Asp Glu Ala Glu Ala Gly
225                 230                 235                 240
Glu Gly Gly Glu Asn
                245

## Description

### Technical Field

The present invention relates to a cell culture medium using fibrin 5 and Zeta polypeptide, a medium kit for cells, a cell culture system, a method for culturing cells, and an iPS cell growth agent.

### Background Art

As a result of recent studies, the possibility of practical use of human pluripotent stem cells such as human ES cells (hESC) or human iPS cells (hiPSC) has been increased in the field of regenerative medicine. These pluripotent stem cells have an infinite proliferative ability and an ability to differentiate into various cells, such as nerve cells, cardiomyocytes, blood cells or retinal cells, and are expected as a therapeutic means for treating intractable disease, lifestyle-related disease, and the like.

Feeder cells act to supply a growth factor for the maintenance culture of pluripotent stem cells, especially human pluripotent stem cells, to stem cells. Thus, human pluripotent stem cells, such as hESC or hiPSC, have heretofore been cultured mainly on a layer of mouse-derived feeder cells (MEF: mouse embryonic fibroblasts). However, the method for culturing the cells under the coexistence of mouse-derived feeder cells has been problematic in terms of safety to living bodies because the feeder cells are mixed into human pluripotent stem cells.

Accordingly, a method involving culturing pluripotent stem cells by using chemically immobilizing MEF on a culture substrate is known as a method for culturing without mixing feeder cells such as MEF, into human pluripotent stem cells (Non Patent Literature 1). However, the culture method has the possibility that unimmobilized cells and detached cells may be mixed, and can by no means completely prevent the mixing of feeder cells. There is also a problem that the quality of feeder cells is not stable.

Meanwhile, means enabling the maintenance culture of human pluripotent stem cells have also been reported for various human cell types (Non Patent Literatures 2 to 5). A method of using, as feeder cells, various human-derived cells, such as fibroblasts, placental cells, bone marrow cells or endometrial cells, without using heterologous cells, has also been reported (Non Patent Literature 6). However, the method of culturing pluripotent stem cells in the presence of human-derived feeder cells has a problem of being labor-intensive in preparing the feeder cells upon culture and a problem that the quality of feeder cells is not stable.

A method involving culture using a medium conditioned with MEF in advance (MEF-CM) is known as a technique for culturing pluripotent stem cells in the absence of feeder cells. For example, Patent Literature 1 describes a method of preparing a cell-conditioned medium comprising a step of culturing mesenchymal stem cells (MSC), descendants thereof, or a cell line derived therefrom in a medium for cell culture and a step of optionally separating the cell culture medium in which the mesenchymal stem cells (MSC) are obtained by proliferating cells obtained by seeding an embryonic stem (ES) cell colony or a descendant thereof in a serum-free medium containing FGF by non-coculture, and describes also that 14-3-3 protein zeta/delta is present in the MSC-conditioned medium. However, these culture methods using conditioned media have problems such as the cumbersomeness of required preparation of the conditioned media and that quality of feeder cells is not stable. 14-3-3 protein zeta/delta is only given as an example of proteins contained in the MSC-conditioned medium.

Meanwhile, the development of media requiring no conditioning with various cells, i.e. chemically defined media, have also been promoted. For example, it has been reported that the addition of a chimera protein of vitronectin and IGF1 enables the culture of ES cells without using feeder cells (Non Patent Literature 7). In addition, media, such as mTeSR1^{R} from STEM CELL Technologies Inc. and STEMPRO (registered trademark) from Life Technologies Inc., are marketed as a media which contains IGF having the effect of promoting growth. However, these media have problems that pluripotent stem cells cannot be stably cultured in the media and that they are poor in proliferative ability therein. Further, although functional proteins in an MEF secretion have been analyzed (Non Patent Literature 8), no useful functional protein has been identified from the secretion of the feeder cells contained in the conditioned medium.

Furthermore, in order to culture pluripotent stem cells, media containing bovine serum, KNOCKOUT (TM) SR (Knockout Serum Replacement: an additive that can be used, instead of serum, to culture ES/iPS cells), and the like are commonly used. However, most of them contain bovine serum-derived proteins, and thus have a risk of causing infectious diseases, such as bovine spongiform encephalopathy (BSE), and a possibility that the bovine serum contains virus, posing a concern of cell contamination by virus. On the other hand, human-derived serum may be used, which is, however, not practical since it has a risk of causing infectious diseases and is difficult to obtain stably and its use is also ethically problematic.

### Prior Art Literatures

### Patent Literatures

Patent Literature 1: JP Patent Publication (Kohyo) No. 2010-500047 A

### Non Patent Literatures

Non Patent Literature 1: Yue X-S, Fujishiro M, Nishioka C, Arai T, Takahashi E, Gong J-S, Akaike T, Ito Y. Feeder cells support the culture of induced pluripotent stem cells even after chemical fixation. PLoS ONE 2012;7:e32707.
Non Patent Literature 2: Hovatta O, Mikkola M, Gertow K et al., A culture system using human foreskin fibroblasts as feeder cells allows production of human embryonic stem cells. Hum Reprod 2003; 18: 1404-1409.
Non Patent Literature 3: Richards M, Fong CY, Chan WK et al., Human feeders support prolonged undifferentiated growth of human inner cell masses and embryonic stem cells. Nat Biotechnol 2002; 20: 933-936.
Non Patent Literature 4: Cheng L, Hammond H, Ye Z et al., Human adult marrow cells support prolonged expansion of human embryonic stem cells in culture. Stem Cells 2003; 21: 131-142.
Non Patent Literature 5: Richards M, Tan S, Fong CY et al., Comparative evaluation of various human feeders for prolonged undifferentiated growth of human embryonic stem cells. Stem Cells 2003; 21: 546-556.
Non Patent Literature 6: Hayato Fukusumi, Yonehiro Kanemura, Development of feeder cell-free culture technique of human ES/iPS cells, Igaku no Ayumi (Progress of Medicine), 2011; 239: 1338-1344.
Non Patent Literature 7: Manton KJ, Richards S, Van Lonkhuyzen D, Cormack L, Leavesley D, Upton Z, A chimeric vitronectin: IGF-I protein supports feeder-cell-free and serum-free culture of human embryonic stem cells, Stem Cells Dev. 2010, 19(9): 1297-1305
Non Patent Literature 8: Chin AC, Fong WJ, Goh LT, Philp R, Oh SK, Choo AB. Identification of proteins from feeder conditioned medium that support human embryonic stem cells. Journal of Biotechnology, 2007; 130:320-8.

### Summary of Invention

### Object to be Solved by the Invention

It is an object of the present invention to develop a culture technique for safely and efficiently culturing pluripotent stem cells, especially a cell culture promoter for safe and efficient culture. In addition, it is another object of the present invention to provide a cell culture medium, a culture kit for cells, a cell culture system, a method for culturing cells, an iPS cell growth agent, and pluripotent stem cells which are obtained by using them.

### Means for Solving the Object

As a result of intensive studies directed towards achieving the aforementioned objects, the present inventors have found that the growth of human pluripotent stem cells can be promoted by adding fibrin 5 and Zeta polypeptide to a medium for pluripotent stem cells, without co-culturing the human pluripotent stem cells with feeder cells, thereby completing the present invention. Specifically, according to the present invention, fibrin 5 and Zeta polypeptide have been identified as factors for promoting the growth of human pluripotent stem cells. Thus a pluripotent stem cell culture technique having a good balance between safety and efficiency is provided.

According to the present invention, the following inventions are provided.
(1) A cell culture medium, which comprises fibrin 5 and Zeta polypeptide.
(2) The cell culture medium according to (1), wherein isolated fibrin 5 and isolated Zeta polypeptide are contained in a basal medium.
(3) The cell culture medium according to (1) or (2), wherein fibrin 5 and Zeta polypeptide are contained at a mass ratio of 1:50 to 50:1.
(4) The cell culture medium according to any one of (1) to (3), wherein the concentration of fibrin 5 contained in the medium is 2 ng/ml or more and 100 ng/ml or less, and/or the concentration of Zeta polypeptide contained in the medium is 2 ng/ml or more and 100 ng/ml or less.
   (4A) The cell culture medium according to any one of (1) to (4), wherein the concentration of fibrin 5 contained in the medium is 2 ng/ml or more and 100 ng/ml or less.
   (4B) The cell culture medium according to any one of (1) to (4) and (4A), wherein the concentration of Zeta polypeptide contained in the medium is 2 ng/ml or more and 100 ng/ml or less.
(5) The cell culture medium according to any one of (1) to (4), wherein the medium does not comprise one to three selected from albumin, serum, and a conditioned medium.
   (5A) The cell culture medium according to any one of (1) to (5), wherein the medium does not comprise albumin.
   (5B) The cell culture medium according to any one of (1) to (5) and (5A), wherein the medium does not comprise serum.
   (5C) The cell culture medium according to any one of (1) to (5), (5A) and (5B) wherein the medium does not comprise a conditioned medium.
(6) The cell culture medium according to any one of (1) to (5), wherein the basal medium is Essential 8 (registered trademark) medium (hereinafter this serum-free medium is referred to also as E8 medium or E8).
   In the cell culture medium of the present invention, the cells are preferably pluripotent stem cells, and the pluripotent stem cells are preferably iPS cells or ES cells.
(7) A medium kit for cells, which comprises fibrin 5 and Zeta polypeptide and a basal medium.
(8) The medium kit for cells according to (7), wherein the basal medium is Essential 8 (registered trademark) medium.
   In the medium kit for cells of the present invention, the cells are preferably pluripotent stem cells, and the pluripotent stem cells are preferably iPS cells or ES cells.
(9) A cell culture system, which comprises (a) the cell culture medium according to any one of (1) to (6) or the medium kit for cells according to (7) or (8) and (b) a cell culture apparatus.
(10) A method for culturing cells, which comprises a step of culturing cells using any one of the cell culture medium according to any one of (1) to (6), the medium kit for cells according to (7) or (8), and the cell culture system according to (9).
(11) The culture method according to (10), wherein the cells are cultured in the absence of feeder cells.
(12) The culture method according to (10) or (11), wherein the cells are pluripotent stem cells.
(13) The culture method according to (12), wherein the pluripotent stem cells are iPS cells or ES cells.
(14) A cell obtained by the cell culture method according to any one of (10) to (13).
(15) An iPS cell growth agent consisting of fibrin 5 and Zeta polypeptide.

### Advantageous Effects of Invention

By culturing cells by using the cell culture medium of the present invention, cells such as pluripotent stem cells can be safely and efficiently proliferated. According to the cell culture medium of the present invention, cells can be cultured without using feeder cells, and thus, the cells can be safely and efficiently cultured.

### Brief Description of Drawings

[Figure 1] Figure 1 shows the results obtained by counting the number of human iPS cells in "E8 medium", "E8 medium conditioned with MEF", or "E8 medium to which various types of protein factors have been added", and then comparing the obtained numbers. In Figure 1, numerical values in parentheses show the concentration of fibrin 5 or Zeta polypeptide.
[Figure 2] Figure 2 shows comparative results regarding the growth efficiency of human iPS cells in "E8 medium", "E8 medium conditioned with MEF", or "E8 medium to which various types of protein factors have been added", which are compared by staining with alkaline phosphatase.
[Figure 3] Figure 3 shows the results obtained by counting the number of human iPS cells in "E8 medium" or "E8 medium to which various types of protein factors have been added", and then comparing the obtained numbers.
[Figure 4] Figure 4 shows comparative results of regarding the growth efficiency of human iPS cells in "E8 medium" or "E8 medium to which various types of protein factors have been added", which are compared by staining with alkaline phosphatase.
[Figure 5] Figure 5 shows an outline configuration of individual constitutional elements of a culture apparatus.
[Figure 6] Figure 6 shows a block diagram of an observation device for an automatic culture apparatus.
[Figure 7] Figure 7 shows a configuration of hardware forming a main part of an automatic culture apparatus.
[Figure 8] Figure 8 shows a configuration of a cell detection system of a cell culture apparatus.
[Figure 9] Figure 9 shows an outline configuration of individual constitutional elements of a culture apparatus.
[Figure 10] Figure 10 shows details of an image processing unit.
[Figure 11] Figure 11 shows an example of a cell extraction treatment conducted by an image processing unit.

### Embodiments for Carrying out the Invention

Hereinafter, the embodiments for carrying out the present invention will be described.

The cell culture medium of the present invention is a medium characterized by comprising fibrin 5 and Zeta polypeptide. By addition of fibrin 5 and Zeta polypeptide, the growth of human pluripotent stem cells can be promoted without using feeder cells or serum. This eliminates the need to perform co-culture with feeder cells and the conditioning of a medium with feeder cells. By using the chemically defined medium which is safe and stable in quality, the culture of pluripotent stem cells can be easily and efficiently performed.

The cell culture medium of the present invention comprises both fibrin 5 and Zeta polypeptide. When both fibrin 5 and Zeta polypeptide are added, their mass ratio is not particularly limited provided that it enables the growth of cells; however, taking into consideration cumbersomeness in additive preparation and cost, they can be added at the following mass ratios. When the amount of fibrin 5 added is not less than the amount of Zeta polypeptide added, the mass ratio of fibrin 5 to Zeta polypeptide is preferably 100:1 or less (for example, 100:1, 99:1, 98:1, 97:1, 96:1, 95:1, 94:1, 93:1, 92:1, 91:1, 90:1, 89:1, 88:1, 87:1, 86:1, 85:1, 84:1, 83:1, 82:1, 81:1, 80:1, 79:1, 78:1, 77:1, 76:1, 75:1, 74:1, 73:1, 72:1, 71:1, 70:1, 69:1, 68:1, 67:1, 66:1, 65:1, 64:1, 63:1, 62:1, 61:1, 60:1, 59:1, 58:1, 57:1, 56:1, 55:1, 54:1, 53:1, 52:1, 51:1, 50:1, 49:1, 48:1, 47:1, 46:1, 45:1, 44:1, 43:1, 42:1, 41:1, 40:1, 39:1, 38:1, 37:1, 36:1, 35:1, 34:1, 33:1, 32:1, 31:1, 30:1, 29:1, 28:1, 27:1, 26:1, 25:1, 24:1, 23:1, 22:1, 21:1, 20:1, 19:1, 18:1, 17:1, 16:1, 15:1, 14:1, 13:1, 12:1, 11:1, 10:1, 9:1, 8:1, 7:1, 6:1, 5:1, 4:1, 3:1, 2:1, or 1:1), more preferably 10:1 or less (for example, 10:1, 9:1, 8:1, 7:1, 6:1, 5:1, 4:1, 3:1, 2:1, or 1:1), still more preferably 5:1 or less (for example, 5:1, 4:1, 3:1, 2:1, or 1:1), particularly preferably 2:1 or less (for example, 2:1 or 1:1); it can be 1:1 as an example. When the amount of Zeta polypeptide added is not less than the amount of fibrin 5 added, the mass ratio of Zeta polypeptide to fibrin 5 is preferably 100:1 or less (for example, 100:1, 99:1, 98:1, 97:1, 96:1, 95:1, 94:1, 93:1, 92:1, 91:1, 90:1, 89:1, 88:1, 87:1, 86:1, 85:1, 84:1, 83:1, 82:1, 81:1, 80:1, 79:1, 78:1, 77:1, 76:1, 75:1, 74:1, 73:1, 72:1, 71:1, 70:1, 69:1, 68:1, 67:1, 66:1, 65:1, 64:1, 63:1, 62:1, 61:1, 60:1, 59:1, 58:1, 57:1, 56:1, 55:1, 54:1, 53:1, 52:1, 51:1, 50:1, 49:1, 48:1, 47:1, 46:1, 45:1, 44:1, 43:1, 42:1, 41:1, 40:1, 39:1, 38:1, 37:1, 36:1, 35:1, 34:1, 33:1, 32:1, 31:1, 30:1, 29:1, 28:1, 27:1, 26:1, 25:1, 24:1, 23:1, 22:1, 21:1, 20:1, 19:1, 18:1, 17:1, 16:1, 15:1, 14:1, 13:1, 12:1, 11:1, 10:1, 9:1, 8:1, 7:1, 6:1, 5:1, 4:1, 3:1, 2:1, or 1:1), more preferably 10:1 or less (for example, 10:1, 9:1, 8:1, 7:1, 6:1, 5:1, 4:1, 3:1, 2:1, or 1:1), still more preferably 5:1 or less (for example, 5:1, 4:1, 3:1, 2:1, or 1:1), particularly preferably 2:1 or less (for example, 2:1 or 1:1); it can be 1:1 as an example.

The concentration of fibrin 5 contained in the medium is not particularly limited, as long as cells are able to grow in the presence of fibrin 5. Taking into consideration addition effect, in general, the lower limit of the concentration is 0.0001 ng/ml, preferably 0.001 ng/ml, more preferably 0.01 ng/ml, even more preferably 0.1 ng/ml, particularly preferably 1 ng/ml, and most preferably 2 ng/ml. Taking into consideration costs, the upper limit of the concentration is 10000 ng/ml, preferably 1000 ng/ml, more preferably 500 ng/ml, even more preferably 400 ng/ml, particularly preferably 300 ng/ml, more particularly preferably 200 ng/ml or less, and even more particularly preferably 100 ng/ml or less.

The concentration of Zeta polypeptide comprised in the medium is not particularly limited provided that it enables the growth of cells; however, taking into consideration addition effect, in general, the lower limit of the concentration is 0.0001 ng/ml, preferably 0.001 ng/ml, more preferably 0.01 ng/ml, still more preferably 0.1 ng/ml, particularly preferably 1 ng/ml, most preferably 2 ng/ml. Taking into consideration cost, the upper limit thereof is 10,000 ng/ml, preferably 1,000 ng/ml, more preferably 500 ng/ml, still more preferably 400 ng/ml, particularly preferably 300 ng/ml, yet more preferably 200 ng/ml or less, more particularly preferably 100 ng/ml or less.

For the amino acid sequence of human fibrin 5, reference can be made to NCBI Reference Sequence: NC_000014.9, and for the amino acid sequence of mouse fibrin 5, reference can be made to NCBI Reference Sequence: NC_000078.6.

The amino acid sequence of human fibrin 5 is shown in SEQ ID NO: 1 of the Sequence Listing.

The amino acid sequence of mouse fibrin 5 is shown in SEQ ID NO: 2 of the Sequence Listing.

For the amino acid sequence of human Zeta polypeptide, reference can be made to NCBI Reference Sequence: NC_000008.11, and for the amino acid sequence of mouse Zeta polypeptide, reference can be made to NCBI Reference Sequence: NC_000081.6.

The amino acid sequence of human Zeta polypeptide is shown in SEQ ID NO: 3 of the Sequence Listing.

The amino acid sequence of mouse Zeta polypeptide is shown in SEQ ID NO: 4 of the Sequence Listing.

Preferably, the cell culture medium to which the fibrin 5 and Zeta polypeptide of the present invention are added is not a conditioned medium.

Preferably, the cell culture medium to which the fibrin 5 and Zeta polypeptide of the present invention are added does not contain serum.

Preferably, the cell culture medium to which the fibrin 5 and Zeta polypeptide of the present invention are added does not contain albumin.

The cell culture medium of the present invention is preferably a medium in which isolated fibrin 5 and isolated Zeta polypeptide are contained in a basal medium, more preferably a medium prepared by adding isolated fibrin 5 and isolated Zeta polypeptide to a basal medium. Fibrin 5 and Zeta polypeptide may be a commercially available one; however, it may also be one isolated from a biomaterial. The protein/polypeptide having the amino acid sequence described in the paragraphs [0018] and [0019] can also be artificially synthesized. Examples of the method for artificial synthesis include a method involving preparation by introducing a gene encoding fibrin 5 or Zeta polypeptide into *Escherichia coli, Bacillus subtilis,* actinomycete, yeast, insect cells, animal cells or plant cells, followed by expression (recombinant protein production method), an cell-free protein synthesis method, and a chemical synthesis method.

Examples of the basal medium include, but are not particularly limited to, BME medium, BGJb medium, CMRL1066 medium, Glasgow MEM medium, Improved MEM Zinc Option medium, IMDM medium, Medium 199 medium, Eagle MEM medium, αMEM medium, DMEM medium, Ham medium, RPMI 1640 medium, Fischer's medium, and a mixed medium thereof as long as it can be used for culture of animal cells. Specific examples include media containing no serum-derived components, to which insulin and transferrin are added, such as CHO-S-SFM II (manufactured by GIBCO BRL), Hybridoma-SFM (manufactured by GIBCO BRL), eRDF Dry Powdered Media (manufactured by GIBCO BRL), UltraCULTURE^{™} (manufactured by BioWhittaker), UltraDOMA^{™} (manufactured by BioWhittaker), UltraCHO^{™} (manufactured by BioWhittaker), and UltraMDCK^{™} (manufactured by BioWhittaker). Essential 8 (registered trademark) (from Life Technologies Inc.), STEMPRO hESC SFM (from Life Technologies Inc.), mTeSR1 (from Veritas Inc.), TeSR2 (from Veritas Inc.), ReproXF (from ReproCell Inc.), and the like developed for use in the culture of human pluripotent stem cells are preferable media. Particularly preferably, the basal medium is Essential 8 (registered trademark) medium.

As described above, the cell culture medium of the present invention can be produced by adding fibrin 5 and Zeta polypeptide to a basal medium. The present cell culture medium can also be used in the form of a cell culture kit, which comprises fibrin 5 and Zeta polypeptide and a basal medium. Specifically, a kit comprising fibrin 5 and Zeta polypeptide and a basal medium, wherein the fibrin 5 and Zeta polypeptide and the basal medium are placed separately, is provided, and when the kit is used, a user adds the fibrin 5 and Zeta polypeptide to the basal medium to prepare the cell culture medium of the present invention, so that the prepared cell culture medium can be used.

Moreover, according to the present invention, a cell culture system comprising (a) the above-described cell culture medium or cell culture kit and (b) a cell culture apparatus is provided.

The cell culture apparatus is not limited, as long as it is an apparatus capable of culturing cells by using the above-described cell culture medium or cell culture kit. For instance, the cell culture apparatuses described in JP Patent Publication (Kokai) No. 2008-92811 A, JP Patent Publication (Kokai) No. 2008-92882 A, and JP Patent Publication (Kokai) No. 2010-148391 A (the content described in the aforementioned publications is incorporated herein as a part of the disclosure of the present description) can be used.

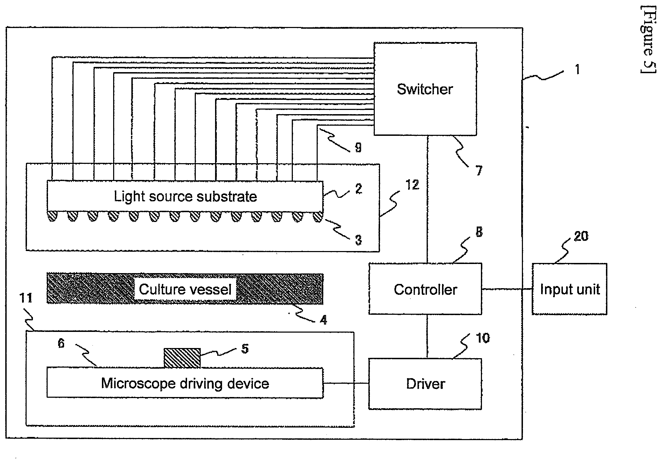

As an example of the present invention, there can be used a cell culture apparatus comprising a culture vessel for culturing cells, an imaging device for taking an image of the cells in the culture vessel, a light source means for applying light to the cells when an image of the cells is taken, and a moving means for moving the microscope, as described in JP Patent Publication (Kokai) No. 2008-92811 A, wherein the cell culture apparatus is characterized in that the light source means has a plurality of light sources, and in that the light source corresponding to the position of the microscope is turned on. An example of the configuration of the cell culture apparatus is shown in Figure 5.

A cell culture apparatus 1 is composed of a culture vessel 4 for culturing cells, a microscope 5 for taking an image of the cells, a microscope driving device 6 for moving the microscope 5, a driver 10 for controlling the microscope driving device 6, a switcher 7 for turning on the power to a specific light source of a plurality of light sources 3 established on a light source substrate, based on the position information of the microscope 5, a light source willing 9 for connecting the switcher 7 with the light sources 3, a light source substrate 2 on which the light sources 3 are aligned, and a controller 8 for controlling these components. Moreover, the culture apparatus is connected with the controller 8, and comprises an input part 20 composed of a trackball or a keyboard. The microscope driving device 6 comprises a driving mechanism having a motor and a ball screw mechanism, and is capable of two-dimensionally moving the microscope 5 via the ball screw mechanism. Furthermore, the ball screw mechanism has a position sensor composed of a rotary encoder or the like that detects the rotation amount of the ball screw, converts it to the position information of the microscope 5 and detects it. The position sensor detects the rotation amount of the ball screw, so that it can two-dimensionally specify the position of the microscope 5. This position information of the microscope 5 is transmitted to the controller 8 via the driver 10. The ball screw mechanism composed of a ball screw having a spiral groove and a sleeve that moves along the groove. The microscope 5 is established on the sleeve. By rotating the ball screw, the sleeve and the microscope 5 can be linearly transferred. The cell culture apparatus comprises two ball screw mechanisms such that they each make a right angle, and as a result, the sleeve and the microscope 5 can be two-dimensionally transferred.

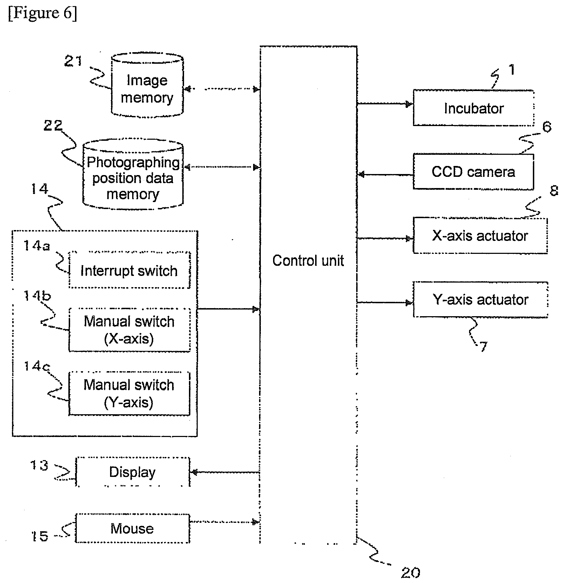

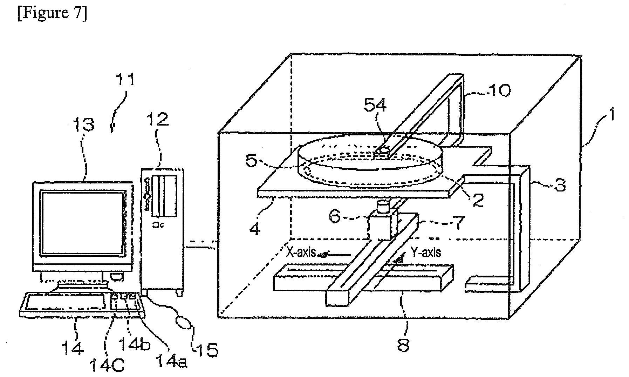

As another example of the present invention, there can be used an automatic culture apparatus comprising a cell incubator, a camera for photographing cells in the incubator, a moving means for relatively moving the position of the camera with respect to the incubator along the photographed surface of the incubator, a memory for storing the image photographed by the camera, a display for displaying the image stored in the memory, and a control means that is formed to be switchable between an automatic photographing mode and a manual photographing mode, as described in JP Patent Publication (Kokai) No. 2008-92882 A, wherein the automatic culture apparatus is characterized in that
the automatic photographing mode has the function of controlling the moving means and allowing the camera to scan with respect to a plurality of small sections that have been obtained by dividing the photographed surface by adjusting it to the previously determined visual field of the camera, then controlling the camera and photographing the surface to be photographed at a small section unit, then storing the photographed image in the memory, then reading out the image stored in the memory, and then allowing the display to display the entire image of the photographed surface, and
(a) the manual photographing mode has the function of controlling the moving means based on the operation command input by the input means and photographing the local image of the photographed surface at any given position by the camera, and at the same time, allowing the display to display the thus photographed local image, or
(b) the manual photographing mode has the function of controlling the moving means and moving the position of the camera based on the position on the display designated by the input means, photographing the local image at the designated position, and allowing the display to display the photographed local image. An example of the configuration of the automatic culture apparatus is shown in each of Figure 6 and Figure 7.

As shown in the perspective view of Figure 7, the present automatic culture apparatus is configured to establish a circular petri dish 2 serving as a cell incubator in an incubator 1, and in the incubator 1, a temperature and a gas concentration such as CO₂ are adjusted, as is publicly known. The petri dish 2 is established on a bedplate 4 supported by a stand 3, and an aperture 5, which does not hinder observation of the bottom surface of the petri dish 2 from below, is established on the bedplate 4. A CCD camera 6 for photographing cells in the petri dish 2 is established below the aperture 5 on the bedplate 4. The CCD camera 6 comprises an objective lens with a high magnification capable of observing cells. It is to be noted that a CMOS camera can be used instead of the CCD camera 6. The CCD camera 6 is mounted on a Y-axis actuator 7 for moving the camera in the Y direction indicated with the illustrated arrow. The Y-axis actuator 7 is mounted on an X-axis actuator 8 for moving the camera in the X direction with the illustrated arrow. In addition, a light source 54 is disposed at a position opposing the CCD camera 6 across the petri dish 2, and the light source 54 is fixed on the CCD camera 6, and it is equipped at the tip of a supporting arm 10. A moving means is configured to relatively move the positions of the CCD camera 6 and the light source 54 to any given positions along the bottom surface of the petri dish 2 by the Y-axis actuator 7 and the X-axis actuator 8. The CCD camera 6, the Y-axis actuator 7, the X-axis actuator 8 and the light source 54 are controlled by a computer 11 such as a personal computer (PC), and an observation device that is a feature of the present invention is composed of these components.

The computer 11 is configured by connecting a computer body 12, a display 13, a keyboard 14 and a mouse 15 serving as input means, and the like. The computer body 12 comprises CPU, memory, I/O, etc., and thus, it is comprises I/O necessary for a general purpose computer.

As shown in Figure 6, the present apparatus comprises a control unit 20 composed of the computer body 12, and the control unit 20 is connected with the incubator 1, the CCD camera 6, the Y-axis actuator 7, the X-axis actuator 8, the display 13, the keyboard 14, and the mouse 15. An interrupt switch 14a, a manual switch (X-axis) 14b, and a manual switch (Y-axis) 14c are established on the keyboard 14. Moreover, an image memory 21 for storing the image photographed by the CCD camera 6 and a photographing position data memory 22 are connected with the control unit 20. Furthermore, the control unit 20 comprises a non-illustrative reading-out means for reading out the image stored in the image memory 21 and allowing the display 13 to display it, and a control means for controlling the photographing by the CCD camera 6 by switching it between an automatic photographing mode and a manual photographing mode.

Details for the operations performed using the present apparatus are as described in paragraphs 0029 to 0053 of JP Patent Publication (Kokai) No. 2008-92882 A.

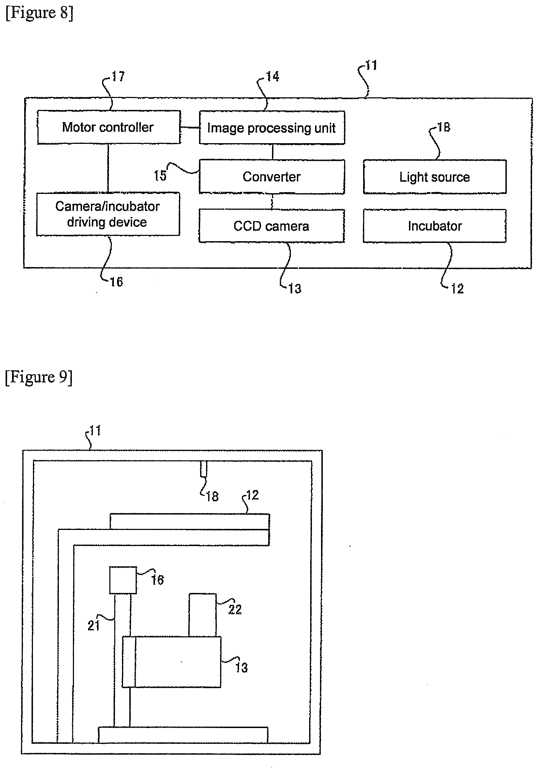

As a further example of the present invention, as described in JP Patent Publication (Kokai) No. 2010-148391 A, there can be used a cell detection system of a cell culture apparatus comprising an incubator means for culturing cells, an image-obtaining means for obtaining the image of the cells from the incubator means, a focus position adjusting means for moving either one of the image-obtaining means and the incubator means and adjusting the focus of the image-obtaining means, and a control means for controlling the image-obtaining means such that images can be obtained at a plurality of focus positions of the image-obtaining means, wherein the cell detection system is characterized in that it comprises an image selection means for selecting an image in which the margin of a cell is seen clearly from the plurality of images obtained at the plurality of focus positions, and an extraction means for performing a subtraction treatment on a first image selected by the image selection means, in which the margin of a cell is seen clearly, and a second image obtained at a position deviated from the focus position of the first image. An example of the configuration of the cell detection system is shown in each of Figure 8 to Figure 11.

As shown in Figure 8, a cell culture apparatus 11 has a box structure in which the internal portion thereof is interrupted from the external space, and in the box structure, the cell culture apparatus comprises an incubator 12 for culturing cells, a CCD camera 13 for photographing the cells in the incubator 12, an image processing unit 14 for processing the image data obtained by the CCD camera 13, a converter 15 for transferring the image data to the image processing unit 14, a camera/incubator driving device 16 for moving the CCD camera 13 or the incubator 12, a motor controller 17 for moving the camera/incubator driving device 16 to any given position, and a light source 18 established above the CCD camera 13. The incubator 12 is molded with a transparent material, and the CCD camera 13 is configured to take a photograph of a transmitted light that has been irradiated from the light source 18 and has then passed through the incubator 12. In the above-described configuration, the CCD camera 13 desirably comprises a CCD device with a resolution of approximately 40,000 pixels, and the light source 18 is not particularly limited, but it is desirably LED in the present Examples.

Figure 9 is a view showing an outline of the arrangement of individual constitutional elements in the culture apparatus 11, and in the illustrative example, an arrangement in a case where the incubator 12 is fixed in the culture apparatus 11 and the CCD camera 13 is moved is shown. As shown in Figure 9, the light source 18 is equipped in the upper portion of the culture apparatus 11. The incubator 12 is disposed on the lower side of this light source 18. The CCD camera 13 comprising an objective lens 22 is disposed on the lower side around the center of the incubator 12. The CCD camera 13 is vertically movably equipped in a moving guide 21, and it moves up and down along the moving guide 21 according to the drive control by the camera/incubator driving device 16 that is allowed to move by the command outputted from a CPU 32, so that it changes the focus position in the vertical direction and then takes a photograph of an image of the cells in the incubator 12. An imaging device in which the CCD camera 13 is combined with the objective lens 22 is desirably configured to be able to take a photograph of a micro area located around the bottom surface around the center of the incubator 12, for example, a micro region with a size of about 1.5 mm (depth) x 2.0 mm (wide).

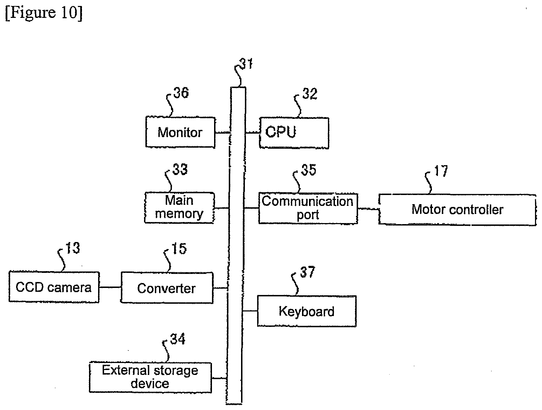

Figure 10 is a view showing details of the image processing unit 14 shown in Figure 8. The image processing unit 14 is composed of a CPU 32 for performing arithmetic processing via a data bus 31, a main memory 33 temporarily used as a storage area by the CPU 32, an external storage device 34 for storing image data or position information, a communication port 35 for communicating with a motor controller 17, a monitor 36 for displaying the image after cell extraction, and a keyboard 37 for receiving the input of the user. In this image processing unit 14, the image from the CCD camera 13 is incorporated into the unit via a converter 15, and various image processing are then carried out.

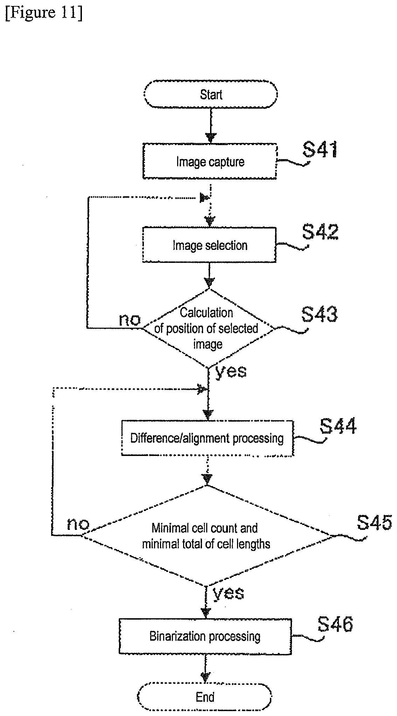

Figure 11 is a flow chart showing an example of a cell extraction processing carried out by the image processing unit. A series of steps shown in Figure 11 are carried out by previously installed software. That is, the steps are carried out by successively drive-controlling the units shown in Figure 8 to Figure 10 by the command outputted from the CPU32, at predetermined time intervals in the cell culture process, for example, repeatedly at a frequency of once/day. As a timer for repeatedly carrying out the series of steps shown in Figure 11 at predetermined time intervals, an output from a clock generator provided in the CPU 32 can be used. Details for the cell extraction treatment shown in Figure 11 are as described in paragraphs 0016 to 0028 of JP Patent Publication (Kokai) No. 2010-148391 A.

According to the present invention, a method for culturing cells, which comprises a step of culturing cells using the above-described cell culture medium, cell culture kit, or cell culture system of the present invention, is provided. Moreover, safe pluripotent stem cells are provided by culturing cells using any one of the cell culture medium, cell culture kit, and cell culture system.

The type of cells cultured in the present invention is not particularly limited, and the cells cultured in the present invention may be either stem cells having both multipotency and self-proliferative ability, or differentiated cells. The cells are preferably stem cells, and more preferably pluripotent stem cells. In the present invention, the "pluripotent stem cells" mean cells that can be cultured *in vitro* and have pluripotency by which the cells can differentiate into all cells constituting a living body. Specific examples of the pluripotent stem cells include embryonic stem cells (ES cells), fetal gonocyte-derived pluripotent stem cells (EG cells: Proc Natl Acad Sci USA. 1998, 95: 13726-13731), testis-derived pluripotent stem cells (GS cells: Nature. 2008, 456: 344-349), and somatic cell-derived induced pluripotent stem cells (induced pluripotent stem cells; iPS cells). Furthermore, animal species, from which cells to be cultured are derived, are not particularly limited, and cells derived from any given mammals and the like can be used. For example, mouse-derived cells, human-derived cells, or the like can be used. It is to be noted that cells can be cultured under ordinary cell culture conditions.

In a preferred aspect of the present invention, cells can be cultured in the absence of feeder cells.

When cells such as pluripotent stem cells are cultured in the absence of feeder cells, the culture can be carried out using a culture vessel coated with a culture substrate serving as a scaffold for cells. The culture substrate serving as a scaffold for cells is not particularly limited, as long as it is used for cell culture. Examples of the culture substrate include gelatin, Matrigel that is an isolated basement membrane component generated from Engelbreth-Holm-Swarm (EHS) mouse sarcoma, placenta matrix, merosin, tenascin, heparin sulfate, chondroitin sulfate, dermatan sulfate, aggrecan, biglycan, thrombospondin, laminin (laminin-511, laminin-111, and laminin-332), fibronectin, vitronectin, collagen, E-cadherin, decorin, a synthetic peptide, a synthetic polymer, and an extracellular matrix derived from MEF or human serum or decidua mesenchymal cells.

Moreover, according to another aspect, cells can also be cultured using the cell culture medium of the present invention, on a culture substrate having a surface that has not been coated with the above-described extracellular matrix.

According to the present invention, cells are provided which are obtained by the above-described method for culturing cells in accordance with the present invention. The cell of the present invention is preferably a pluripotent stem cell, more preferably an iPS cell or an ES cell. Since the circumstance is present that it is impossible or impractical to identify the structure per se of the cell of the present invention without resort to its production method, the cell of the present invention is defined by the cell culture method (i.e. the culture method using a cell culture medium comprising fibrin 5 and Zeta polypeptide).

As demonstrated in the following Examples, it has been found by the present invention that iPS cells can be cultured using the cell culture medium of the present invention comprising fibrin 5 and Zeta polypeptide to promote the growth of iPS cells. Thus, according to the present invention, an iPS cell growth agent is also provided which consists of a combination of fibrin 5 and Zeta polypeptide. The iPS cell growth agent consisting of a combination of fibrin 5 and Zeta polypeptide can be used, for example, in the form of an iPS cell culture medium by adding it to a basal medium as described above.

Further, according to the present invention, a combination of fibrin 5 and Zeta polypeptide for use as a cell culture medium is provided.

Moreover, according to the present invention, the use of fibrin 5 and Zeta polypeptide for producing a cell culture medium is provided.

The present invention will be more specifically described in the following examples. However, these examples are not intended to limit the scope of the present invention.

### Examples

### (1) Detection of protein in conditioned serum-free medium

A conditioned medium (CM) was prepared from a serum-free medium, using mouse embryonic fibroblasts (MEF cells) that had been inactivated by being treated with mitomycin. The mouse embryonic fibroblasts (MEF cells) that had been inactivated by the mitomycin treatment were inoculated at a cell density of approximately 500,000 cells/a dish with a diameter of 60 mm in a medium for MEF (DMEM medium containing 10% FBS). The cells were cultured for at least 16 hours, and thereafter, the resulting cells were washed with PBS(-), and then with an Essential 8^{™} medium (Invitrogen). After that, the medium was replaced with a fresh serum-free medium of the same type.

After the replacement of the medium, the medium was incubated for 24 hours in a CO₂ incubator (37°C, 5% CO₂ concentration) and then recovered to provide a conditioned medium.

An unconditioned serum-free medium and a conditioned serum-free medium were subjected to comparative analysis by proteome analysis. Acetone (20 ml) cooled to -80°C was added to 5 ml of each medium, which was then allowed to stand at -80°C overnight and centrifuged at 15,000 rpm and 4°C for 30 minutes, followed by removing the supernatant. The precipitate was air-dried and then dissolved in 250 µL of a sample buffer (a 30 mM Tris-HCl buffer solution containing 7M urea, 2M thiourea, and 4% CHAPS (pH8.5)). The solution (170 µL) was supplied to DiNa-2A two-dimensional autoinjector system (from KYA Technologies Corporation, JPN)). The gel obtained by two-dimensional electrophoresis was stained with Sypro Ruby to compare the spots. The spot specifically detected in the conditioned medium was cut out and subjected to reduction with dithiothreitol, Cys-SH group alkylation with iodoacetamide, and subsequent enzymatic digestion with trypsin (37°C, overnight). The enzyme-digested solution was recovered, subjected to exsiccation under reduced pressure by SpeedVac, and then re-dissolved in 10 µL of 0.1% formic acid/2% acetonitrile. This solution was centrifuged (15,000 g, 10 minutes, room temperature), and the supernatant was supplied to LCMS-IT-TOF (Shimadzu liquid chromatograph mass spectrometer) and subjected to protein identification by MS/MS ion search. Search was carried out using Mascot (Matrix Science Inc.) as a database search engine and using NCBI database.

As a result, extracellular matrix proteins such as collagen, fibronectin and osteonectin, as well as fibrin 5, Zeta polypeptide and the like were detected.

### (2) Protein addition test 1

### (2-1) Cases of unconditioned serum-free medium or conditioned serum-free medium

Human iPS cells (201B7) used in the experiment were obtained from iPS Academia Japan, Inc. Human iPS cells were prepared by carrying out a maintenance culture on a plastic culture dish, on which mouse embryonic fibroblasts inactivated by a mitomycin treatment were plated as feeder cells. A medium prepared by adding KNOCKOUTTM SR (final concentration: 20%), 0.1 mM NEAA (non-essential amino acids), 2 mM L-glutamine, 5 ng/ml human basic FGF and 0.1 mM 2-mercaptoethanol to D-MEM/F12 (Sigma D6421) was used. The cells were cultured at 37°C in a CO₂ incubator. Subculture was carried out every 6 to 7 days, and using a dissociation solution (collagen solution), a colony of human iPS cells was removed from a feeder layer, and about 20 to 50 small masses were then obtained by a pipetting operation. The obtained small masses were plated on a fresh feeder cell layer. Human iPS cells, which had been subjected to a maintenance culture on feeder cells, were removed using a dissociation solution, and about 20 to 50 small masses were then obtained by a pipetting operation. The masses were centrifuged at 300 rpm for 5 minutes to recover iPS cells, and MEF was then removed by incubation of the cells on a gelatin-coated culture dish for 30 minutes. The resulting cells were divided into 4 portions, and the one portion was then inoculated on a culture dish coated with Matrigel (registered trademark) (BD). As a medium, a serum-free medium (E8 medium) that had not been conditioned with MEF was used, and it was compared with an E8 medium (E8-CM) that had been conditioned with MEF.

The results regarding the comparison of proliferative ability are shown in Table 1. It was found that the proliferative efficiency of human iPS cells was improved in the conditioned medium, in comparison to in the unconditioned medium.

### (2-2)

200 µg/ml Bovine collagen I (Nitta Gelatin Inc.), human fibronectin (BD Bioscience Inc.), or mouse osteonectin (R&D Systems Inc.) or 2 ng/ml or 100 ng/ml human fibrin 5 (USCN Life Science Co., Ltd.) or human Zeta polypeptide (ATGEN Co., Ltd.) was added to a serum-free medium (E8 medium) to examine the influence thereof on the growth of human iPS cells, as in the case of (2-1) above.

The results of comparing proliferative ability are shown in Table 1. -, +, and ++ in Table 1 were determined according to the following criteria.
-: no growth-promoting effect was observed compared to the case of using E8 medium.
+: a weak growth-promoting effect was observed compared to the case of using E8 medium.
++: a strong growth-promoting effect was observed compared to the case of using E8 medium.

Fibrin 5 and Zeta polypeptide were observed to have the effect of promoting the growth of human iPS cells. On the other hand, collagen I, fibronectin, or osteonectin was not observed to have such a growth-promoting effect.

**[Table 1]**

| Conditioning/Additive | Addition Concentration | Increase (%) in Growth Efficiency Relative to E8 Medium | Growth-promoting Effect |
|---|---|---|---|
| Conditioned (E8-CM) | - | 477 | ++ |
| Collagen I | 200 µg/ml | 0 | - |
| Fibronectin | 200 µg/ml | 0 | - |
| Osteonectin | 200 µg/ml | 0 | - |
| Fibrin 5 | 2ng/ml | 63 | + |
| | 100ng/ml | 117 | + |
| Zeta Polypeptide | 2ng/ml | 50 | + |
| | 100ng/ml | 143 | + |

### (3) Protein addition test 2

The influence thereof on the growth of human iPS cells was examined as in the case of (2-1) above except for using the medium described below as a medium and coating the culture dish with Matrigel (registered trademark) (BD).

Fibrin 5 (also indicated with FLN5) and Zeta polypeptide (also indicated with ζ) were each added in a concentration of 2 ng/ml or 100 ng/ml to a serum-free medium (E8 medium) to examine the influence thereof on the growth of human iPS cells as in the case of (2-1) above.

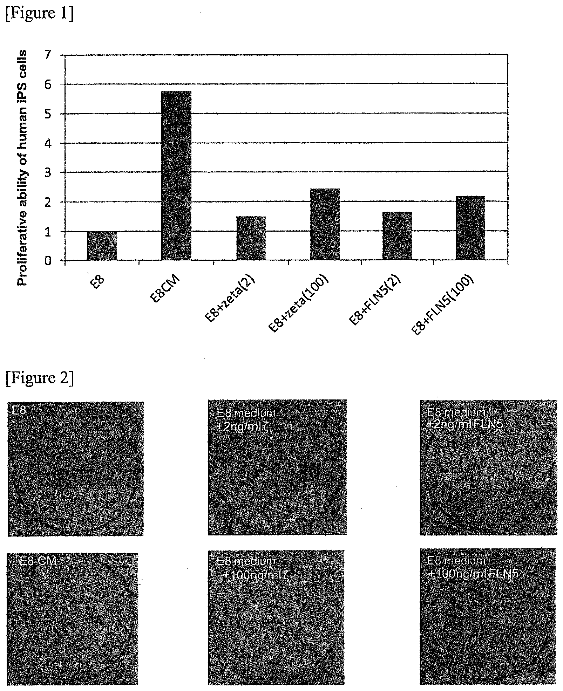

To compare the proliferative ability of cells, the number of cells was counted. The number of cells in the case of using E8 medium was defined as 1, and the results of determining the ratio of the number of cells in the case of using another medium to that in the case of using E8 medium are shown in Figure 1.

The results of staining cells with alkaline phosphatase are shown in Figure 2. The addition of both fibrin 5 and Zeta polypeptide enhanced the effect of promoting growth compared to the addition of any one thereof.

### (4) Protein addition test 3

The influence thereof on the growth of human iPS cells was examined as in the case of (2-1) above except for using the medium described below as a medium and coating the culture dish with iMatrix (registered trademark) (Nippi, Inc.).

2 ng/ml Fibrin 5 or Zeta polypeptide or 2 ng/ml each of both fibrin 5 and Zeta polypeptide were added to a serum-free medium (E8 medium) to examine the influence thereof on the growth of human iPS cells as in the case of (2-1) above.

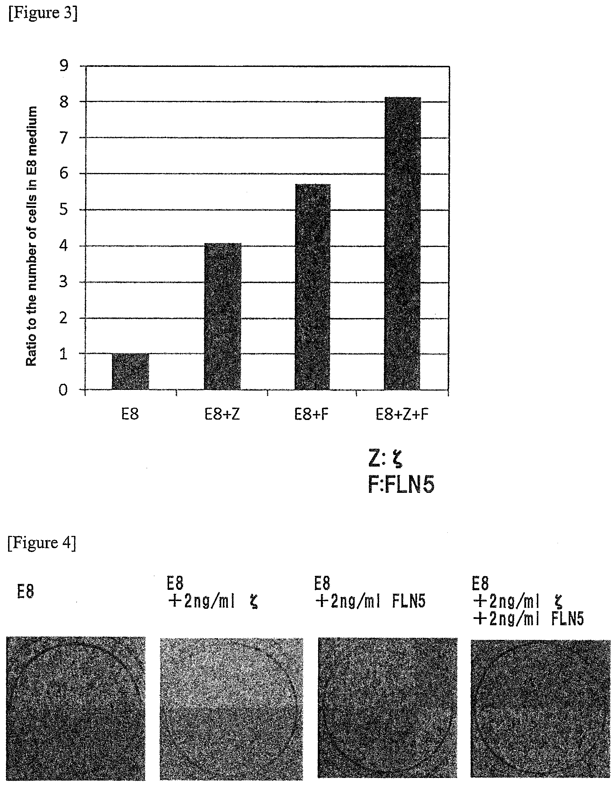

To compare the proliferative ability of cells, the number of cells was counted. The number of cells in the case of using E8 medium was defined as. 1, and the results of determining the ratio of the number of cells in the case of using another medium to that in the case of using E8 medium are shown in Figure 3. The results of staining cells with alkaline phosphatase are shown in Figure 4. The addition of both fibrin 5 and Zeta polypeptide enhanced the effect of promoting growth compared to the addition of any one thereof.

## Claims

1. A cell culture medium, which comprises fibrin 5 and Zeta polypeptide.

2. The cell culture medium according to claim 1, wherein isolated fibrin 5 and isolated Zeta polypeptide are contained in a basal medium.

3. The cell culture medium according to claim 1 or 2, wherein fibrin 5 and Zeta polypeptide are contained at a mass ratio of 1:50 to 50:1.

4. The cell culture medium according to any one of claims 1 to 3, wherein the concentration of fibrin 5 contained in the medium is 2 ng/ml or more and 100 ng/ml or less, and/or the concentration of Zeta polypeptide contained in the medium is 2 ng/ml or more and 100 ng/ml or less.

5. The cell culture medium according to any one of claims 1 to 4, wherein the medium does not comprise one to three selected from albumin, serum, and a conditioned medium.

6. The cell culture medium according to any one of claims 1 to 5, wherein the basal medium is Essential 8 (registered trademark) medium.

7. A medium kit for cells, which comprises fibrin 5 and Zeta polypeptide and a basal medium.

8. The medium kit for cells according to claim 7, wherein the basal medium is Essential 8 (registered trademark) medium.

9. A cell culture system, which comprises (a) the cell culture medium according to any one of claims 1 to 6 or the medium kit for cells according to claim 7 or 8 and (b) a cell culture apparatus.

10. A method for culturing cells, which comprises a step of culturing cells using any one of the cell culture medium according to any one of claims 1 to 6, the medium kit for cells according to claim 7 or 8, and the cell culture system according to claim 9.

11. The culture method according to claim 10, wherein the cells are cultured in the absence of feeder cells.

12. The culture method according to claim 10 or 11, wherein the cells are pluripotent stem cells.

13. The culture method according to claim 12, wherein the pluripotent stem cells are iPS cells or ES cells.

14. A cell obtained by the cell culture method according to any one of claims 10 to 13.

15. An iPS cell growth agent consisting of fibrin 5 and Zeta polypeptide.
